(19) Europäisches Patentamt
European Patent Office
Office européen des brevets

(11) Publication number: **0 309 424 A2**

(12) # EUROPEAN PATENT APPLICATION

(21) Application number: **88830376.5**

(22) Date of filing: **19.09.88**

(51) Int. Cl.[4]: **C 07 D 451/10**
C 07 D 211/46,
C 07 D 295/18,
C 07 D 295/20,
C 07 D 241/08,
C 07 D 243/08, A 61 K 31/46,
A 61 K 31/445,
A 61 K 31/495,
C 07 C 129/12, C 07 C 123/00

(30) Priority: **21.09.87 IT 2197787**

(43) Date of publication of application:
**29.03.89 Bulletin 89/13**

(84) Designated Contracting States:
**AT BE CH DE ES FR GB GR IT LI LU NL SE**

(71) Applicant: **ISTITUTO DE ANGELI S.p.A.**
**Via Serio, 15**
**I-20139 Milano (IT)**

(72) Inventor: **Cereda, Enzo**
**Via Romagnolo, 2A**
**Tortona (Alessandria) (IT)**

**Donetti, Arturo**
**Viale Romagna, 4**
**Milano (IT)**

**Turconi, Marco**
**Via Gramsci, 20**
**Voghera (Pavia) (IT)**

**Schiavi, Giovanni Battista**
**Via Montello, 7**
**Asola (Mantova) (IT)**

**Micheletti, Rosamaria**
**Via Borgospesso, 25**
**Milano (IT)**

**Giachetti, Antonio**
**Via Guerrini, 3**
**Milano (IT)**

(74) Representative: **Aimi, Luciano et al**
**c/o Società Italiana Brevetti S.p.A. Via Carducci 8**
**I-20123 Milano (IT)**

(54) **New amidino derivatives.**

(57) New pharmacologically active amidino derivatives as muscarinic receptor blocking agents which are useful for the treatment of gastrointestinal disorders of the following formula

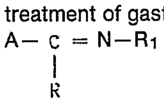 (I)

wherein
R represents hydrogen atom, $C_{1-5}$ alkyl, aryl, an amino group optionally substituted by one or two $C_{1-5}$ alkyl;
$R_1$ represents hydrogen atom, $C_{1-8}$ alkyl, aralkyl;
A represents
$C_{1-5}$ alkyl substituted by 2 or 3 radicals, which may be identical or different from each other, selected from aryl, cycloalkyl, carboxamide;
or a saturated 6-membered heterocyclic ring containing one nitrogen atom N-substituted by $COR_2$ where $R_2$ is a methyl substituted by 2 or 3 radicals, which may be identical or different from each other, selected from aryl, cycloalkyl, hydroxy;
or

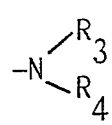

where $R_3$ is hydrogen atom and $R_4$ is $C_{1-5}$ alkyl substitued by 2 or 3 radicals, which may be identical or different from each other, selected from aryl, cycloalkyl, carboxamide, or by hydroxy optionally esterified with a cycloalkylcarboxylic acid, or $R_3$ and $R_4$, together with the nitrogen atom to which they are attached, form a saturated 5- to 7-membered heterocyclic ring, optionally containing one or two nitrogen atoms or an endocyclic carboxamido group optionally comprising in its inside $-CH_2-CH_2-$ or

EP 0 309 424 A2

$-CH_2-CH_2-$ or 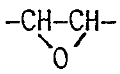 ,

being the heterocyclic ring substituted by a $C_{1-5}$ alkyl substituted in turn by 2 or 3 radicals, which may be identical or different from each other, selected from $C_{1-5}$ alkyl, aryl, cycloalkyl or hydroxy, or byarylalkylene, or by $-OR_5$ where $R_5$ is $-OCR_6$ where $R_6$ is methyl substituted by 2 or 3 radicals, which may be identical or different from each other, selected from a saturated or unsaturated $C_{1-5}$ alkyl, aryl, cycloalkyl, hydroxy $C_{1-5}$ alkyl, hydroxy, or unsaturated 6-membered heterocyclic ring, provided that at least one between R and A is amino group or

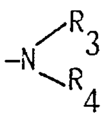 .

The tautomers and the acid addition salts of these compounds, as well as the process for the preparation of the compounds of formula (I) and pharmaceutical compositions containing them are also described.

## Description

# NEW AMIDINO DERIVATIVES

The present invention relates to novel pharmacologically active amidino derivatives, to the process for their preparation and to the pharmaceutical compositions containing them. The new compounds are muscarinic receptors blocking agents useful for the treatment of gastrointestinal disorders.

It is known that administration of muscarinic receptor blocking agents gives rise to a number of pharmacological effects like decreased gastrointestinal motility, inhibition of acid secretion, dry mouth, mydriasis, urinary incontinence, decreased sweating, tachycardia. Furthermore, antimuscarinic agents with tertiary amine structures may give rise to central effects owing to their penetration across blood-brain barrier. The lack of selectivity among these actions makes it difficult to address therapy in one specific indication and this prompted chemical modification of these agents. One of these modifications consists in quaternization of the tertiary amine function to prevent penetration into the brain. The quaternary drugs lack prominent central actions and additionally show a selective greater effect on the gastrointestinal tract, while displaying a minor incidence of side-effects. However their major drawback is the poor and unreliable absorption for oral administration unexploitable for therapeutical purpose.

We have now synthetized, and this is the object of the present invention, a new class of amidino derivatives endowed with a strong antimuscarinic activity which show a further enhanced activity on the gastrointestinal tract associated with a lack of central and peripheral effets such as mydriasis, tachycardia and dry mouth.

Moreover the new amidino derivatives are potentially useful as therapeutically active agents in the management of gastrointestinal motility disorders, such as the spastic condition of the gut, functional diarreha, constipation, irritable bowel syndrome, cardiospasm, pylorospasm, gastro-oesophageal reflux, petic ulcer disease, spasm of the urinary and biliary tracts and urinary incontinence.

The compounds object of the present invention have the general formula (I)

$$A-\underset{\displaystyle R}{\overset{}{\underset{|}{C}}}=N-R_1 \quad (I)$$

wherein

R represents hydrogen atom, $C_{1-5}$ alkyl, aryl, an amino group optionally substituted by one or two $C_{1-5}$ alkyl;

$R_1$ represents hydrogen atom, $C_{1-8}$ alkyl, aralkyl;

A represents

$C_{1-5}$ alkyl substituted by 2 or 3 radicals, which may be identical or different from each other, selected from aryl, cycloalkyl, carboxamide;

or a saturated 6-membered heterocyclic ring containing one nitrogen atom N-substituted by $COR_2$ where $R_2$ is a methyl substituted by 2 or 3 radicals, which may be identical or different from each other, selected from aryl, cycloalkyl, hydroxy;

or

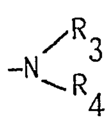

where $R_3$ is hydrogen atom and $R_4$ is $C_{1-5}$ alkyl substitued by 2 or 3 radicals, which may be identical or different from each other, selected from aryl, cycloalkyl, carboxamide, or by hydroxy optionally esterified with a cycloalkylcarboxylic acid, or $R_3$ and $R_4$, together with the nitrogen atom to which they are attached, form a saturated 5- to 7-membered heterocyclic ring, optionally containing one or two nitrogen atoms or an endocyclic carboxamido group optionally comprising in its inside $-CH_2-CH_2-$ or

$-CH_2-CH_2-$ or 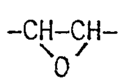 ,

being the heterocyclic ring substituted by a $C_{1-5}$ alkyl substituted in turn by 2 or 3 radicals, which may be identical or different from each other, selected from $C_{1-5}$ alkyl, aryl, cycloalkyl or hydroxy, or byarylalkylene, or by $-OR_5$ where $R_5$ is $-OCR_6$ where $R_6$ is methyl substituted by 2 or 3 radicals, which may be identical or different from each other, selected from a saturated or unsaturated $C_{1-5}$ alkyl, aryl, cycloalkyl, hydroxy $C_{1-5}$ alkyl, hydroxy, or unsaturated 6-membered heterocyclic ring, provided that at least one between R and A is amino group or

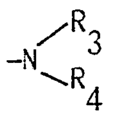 .

For pharmaceutical use the compounds (I) are used as such or under the form of tautomers or of physiologically compatible acid addition salts.

The term "acid addition salts" includes salts with inorganic or organic acids. The physiologically compatible acids used for salification include, for example, salts formed with maleic, citric, hydrochloric, tartaric, hydrobromic, fumaric, nitric, sulphuric, methanesulphonic or hydroiodic acid.

Although the double bond of the amidine groupments is indicated in the general formula (I) as present in a particular position, other tautomeric forms are also possible. The present invention includes therefore such tautomeric forms as regards both the compounds and the manufacturing processes.

Some compounds of formula (I), according to the present invention, contain one or two asymmetric carbon atoms. The compounds may therefore occur as enantiomers of (+) and (-) type, as diastereoisomers or mixture of them. The present invention includes therefore both the individual isomers and the mixture thereof. It has to be understood, that when mixture of optical isomers are present, they may be separated according to the classic resolution methods based on their different physico-chemical properties, e.g. by fractional crystallization of their acid addition salts with a suitable optically active acid or by the cromatographic separation with a suitable mixture of solvents.

In the present invention the term "lower alkyl" means a straight or branched alkyl group having preferably 1 to 5 carbon atoms. The term "aryl" preferably means phenyl. The term "cycloalkyl" preferably means that the ring has 3 to 7 carbon atoms. The term "a saturated 6-membered heterocyclic ring" preferably means piperidine. When $R_3$ and $R_4$ together with the nitrogen atom to which they are attached form a saturated 5- to 7-membered heterocyclic ring which may contain one or two nitrogen or an endocyclic carboxamido group, it may be pirrolidine, piperazine, homopiperazine,2-oxopiperazine or piperidine which may optionally contain in its inside $-CH_2-CH_2-$ or

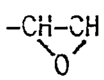

such as desmethyltropane or 6,7-epoxydesmethyltropane. The term "cycloalkyl carboxylic acid" preferably means cyclohexane carboxylic acid. The term "arylalkylene" prefe rably means diphenylmethylene group. The term "hydroxy $C_{1-5}$ alkyl" preferably means hydroxymethyl. The term "unsaturated 6-membered heterocyclic ring"preferably means pyridine.

The compounds of general formula(I) of the present invention may for example be prepared by the following processes, well known in their general lines to the technicians of the branch.

a) Compounds of general formula (I) wherein R is hydrogen and A is

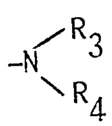

in which $R_3$ and $R_4$ are as herein before defined may be obtained by reacting a compound of formula (II)

A - H (II)

in which A is as above defined, with an addition salt of a compound of formula (III)

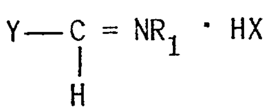 (III)

in which $R_1$ is as hereinbefore defined, Y is a leaving group such as halogen, lower alkoxy, phenoxy, dichlorophosphoryl, preferably ethoxy or methoxy, and HX is a mineral or organic acid such as hydrochloric, hydrobromic, hydroiodic, sulphuric, nitric, tetrafluoboric, alkylsulphonic, arylsulphonic, thiocyanic, preferably

hydrochloric. The reaction is conveniently carried out in a polar solvent such as methanol, ethanol, acetonitrile, acetone, ethylacetate or a mixture of them. The reaction temperature is generally kept between 10°C and 70°C, preferably at room temperature.

The same compounds may be also obtained by desulphurizing a thiourea of general formula (IV)

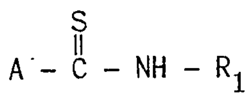
(IV)

in which A and $R_1$ are as above defined,with Raney/Nickel or $H_2O_2$ in an appropriate solvent selected from dichloromethane, chloroform, methanol, ethanol, water or a mixture of them. The process is conveniently carried out at a temperature between 10°C and 70°C, preferably at room temperature.

The thiourea derivatives of general formula (IV), used as starting material in the above process, may be prepared by reacting a compound of formula (II) or its hydrochloric acid addition salt with an isothiocyanate of general formula (V)

$R_1 - N = C = S$ (V)

where $R_1$ is as hereinbefore defined, or with ammonium thiocyanate. The reaction is carried out in solvents such as water, methanol, ethanol, tetrohydrofurane, acetone,preferably tetrahydrofurane or water, or without solvents, at a temperature ranging from 0°C to 200°C, prefarably at room temperature or at 100°C, or at the melting point of the mixture of the reagents.

b) Compounds of general formula (I), wherein R is $C_{1-5}$ alkyl and A is

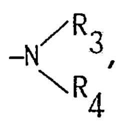

in which $R_3$ and $R_4$ are as hereinbefore defined, may be prepared by reacting a compound of general formula (II) with a compound of formula (VI)

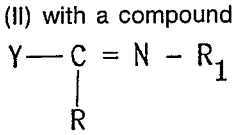
(VI)

in which R, $R_1$ and Y are as hereinbefore defined. Compounds of formula (II) and (VI) may be conveniently used either as free bases or as addition salts with mineral or organic acid of formula HX as hereinbefore defined. The reaction is conveniently carried out in polar solvents such as methanol, ethanol, acetonitrile, acetone, ethylacetate or a mixture of them at a temperature between 10°C and 70°C, preferably at room temperature.

When also $R_1$ in the compounds of general formula (I) is a hydrogen atom,the compounds of formula (II) are reacted with a cyano alkyl derivative of formula (VII)

$R-CN$ (VII)

in which R is as above defined, in the presence of a Lewis acid such as aluminium chloride, zinc chloride, iron III chloride, tin IV chloride, triphenyltin chloride, preferably aluminium chloride. The reaction is optionally carried out in an inert, high-boiling, halogenated hydrocarbon such as chlorobenzene, tetrachloroethane or without any solvents as a molten mass. The reaction temperature ranges from 50°C to 200°C, preferably at the boiling point of the selected solvent.

c) Compounds of general formula (I) wherein R is an amino group optionally substituted by one or two $C_{1-5}$ alkyl groups or by an aryl group and A is

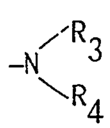

wherein $R_3$ and $R_4$ are as herein before defined, and in particular when R is an unsubstituted amino group and $R_1$ is hydrogen may be obtained by reacting the compound of formula (II) in the form of its addition salt with a mineral or organic acid of formula HX as herein before defined,with cyanamide in the absence of any solvents and at a temperature ranging from 70°C to 160°C, preferably at 100°C, or in the presence of a protic solvent

such as methanol, ethanol, water or a mixture of them at a temperature between 50° C and 100° C, preferably at the boiling point of the solvent. These compounds may be also prepared by reacting a compound of formula (II) with a reactive compound of formula (VIII)

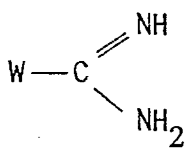
(VIII)

where W is a suitable leaving group selected from 3,5-dimethylpyrazol-1-yl, $C_{1-5}$ thioalkyl, preferably thiomethyl, or sulphonyl. Compounds of formula (VIII), if necessary, may be reacted in the form of their addition salts with mineral or organic acid of formula HX, as hereinbefore defined. The reaction is carried out in polar solvents such as methanol, ethanol, acetonitrile, acetone, water or mixture of them at a temperature ranging from 20° C to 100° C, preferably at the reflux temperature of the selected solvent.

Another way of preparation foresees the reduction of nitroguanidine derivative of general formula

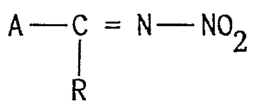
(IX)

wherein A and R are as hereinbefore defined,with hydrogen or a hydrogen donor such as formic acid, acetic acid, ammonium formate, cyclohexene, cyclohexadiene, preferably formic acid in the presence of a suitable catalyst, preferably Pd/C or Pd black, in the presence or in the absence of a suitable solvent such as formic acid, water, methanol, ethanol or mixtures of them, preferably formic acid. The same reduction may be conveniently carried out with reducing agents such as titanium III chloride in hydroalcoholic solvents or tin II chloride in diluted formic acid. The reaction is performed at a temperature ranging from 10° C to 100° C, preferably at 40° C. Compounds of formula (IX), used as starting material in the above process, may be prepared by reacting a compound of formula (II) with a reactive intermediate of general formula (X)

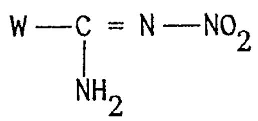
(X)

wherein W is as hereinbefore defined. The reaction is carried out in solvents such as methanol, chloroform, methylene chloride or mixtures of them, preferably in a 1:1 mixture of methanol and methylene dichloride, at a temperature ranging from 10° C to 80°, preferably at room temperature.

When in particular at least one between R and $R_1$ is different from hydrogen or from an unsubstituted amino group, they may be prepared by reacting a compound of general formula (XI)

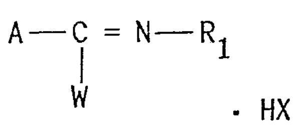
(XI)

where A, $R_1$, HX, W are as hereinbefore defined, with a primary or secondary alkyl amino derivative. The reaction is carried out in a polar solvent such as methanol, ethanol, acetonitrile, acetone, water or a mixture of them at a temperature ranging from 0° C to 100° C, preferably at 40° C. Compounds of formula (XI), used as starting material in the above process, are conveniently prepared by reacting a thiourea derivative of formula (IV) with a sulphur alkylating agent selected from methyliodide, ethyliodide or dimethyl sulphate in the presence of a solvent selected from methanol, ethanol, acetone, acetonitrile, water or a mixture of them at a temperature between 20° C and 100° C, preferably at the reflux temperature of the selected solvent.

d) Compounds of the general formula (I) where A is a $C_{1-5}$ alkyl substituted by 2 or 3 radicals, which may be identical or different from each other, selected from aryl, cycloalkyl, carboxamide, or it is a saturated 6-membered heterocyclic ring, containing one nitrogen atom, N-substituted by $COR_2$ where $R_2$ is a methyl group substituted by 2 or 3 radicals, which may be identical or different from each other, selected from aryl, cycloalkyl or hydroxy and R is an amino group optionally substituted by one or two $C_{1-5}$ alkyl or by an aryl, may be obtained by reacting a cyano derivative or general formula (XII)

A—CN (XII)

in which A is as above defined, with a substituted or unsubstituted ammonium salt of general formula (XIII)

R • HX (XIII)

wherein R and HX are as hereinbefore defined, or with a substituted or unsubstituted thiourea of general formula (XIV)

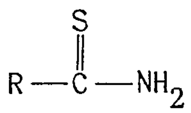

(XIV)

The reaction is simply carried out by mixing the two reagents and heating the mass above its melting point at a temperature between 50° C and 200° C, preferably between 100° C and 200° C. The same compounds may be also prepared by reacting a compound of formula (XII) with a compound of formula (XV)

R—H (XV)

wherein R is as hereinbefore defined, in the presence of a Lewis acid such as aluminium chloride, zinc chloride, iron III chloride, tin IV chloride, triphenyltin chloride, preferably aluminium chloride. The reaction is optionally carried out in an inert, high-boiling, halogenated hydrocarbon such as dichlorobenzene, tetrachloroethane or without any solvent as a molten mass. The reaction temperature ranges from 50° C to 200° C, preferably at the boiling point of the selected solvent. However in order to obtain the same compounds it is exploitable the reaction of an imidate or imidoyl derivative of formula (XVI)

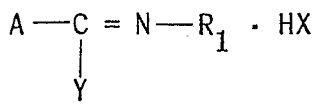

(XVI)

where A, Y, $R_1$ and HX are as hereinbefore defined,with a compound of formula (XV). The reaction is conveniently carried out in a solvent such as methanol, ethanol, acetonitrile, acetone or a mixture of them at a temperature ranging from 0° C to 80° C, preferably at room temperature.

The compounds of general formula (I) prepared according to the processes as above described may optionally be converted with inorganic or organic acids into the corresponding physiologically compatible acid addition salts, for example, by conventional methods such as by reacting the compounds as bases with a solution of the corresponding acid in a suitable solvent. Particularly preferred acids include for example hydrochloric, hydrobromic, sulphuric or methansulphonic acid.

A group of the preferred compounds, according to the present invention, for their better activity as muscarinic receptor blocking agents, is the one formed by the compounds of general formula (I) wherein R is a hydrogen atom, a $C_{1-5}$ alkyl or an unsubstituted amino group, $R_1$ is a hydrogen atom or a $C_{1-5}$ alkyl and A is a radical selected from noratropine, norscopolamine, N- -[(2-hydroxy-2-cyclohexyl-2-phenyl)-ethyl]-piperazine, N-[(2-hydroxy-2-cyclohexyl-2-phenyl)ethyl]-homopiperazine, N-[(2-hydroxy-2,2-diphenyl)-ethyl]-piperazine, benzeneacetic acid α-phenyl- 4-piperidinyl ester, benzeneacetic acid α-cyclohexyl- 4-piperidinyl ester, benzeneacetic acid α-hydroxy-α-phenyl- 4-piperidinyl ester or benzeneacetic acid α-methyl -α-phenyl-4-piperidinyl ester.

As already mentioned hereinbefore the new compounds of formula (I) according to the present invention, have interesting pharmacological properties owing to their ability to antagonize the physiological muscarinic effects in warm blooded animals. Therefore the new compounds are commercially viable in the prevention and in the treatment of motility disorders wherein muscarinic receptors are involved, particularly for spastic condidions of the gastrointestinal tract. and irritable bowel syndrome.

The following tests show that the compounds according to the invention have favourable characteristics in this respect.

## PHARMACOLOGY

ANTIMUSCARINIC ACTIVITY (in vitro binding studies)

Antimuscarinic activity was examined by studying the displacement of $^3$H-pirenzepine from cerebral cortex homogenate according to the procedure reported below:
The cerebral cortex donors were male CD-COOBBS rats, 220-250 g body weight. The homogenization process was carried out in a Potter-Evelhjem apparatus in the presence of $Na^+/Mg^{++}$ HEPES buffer; pH 7.4 (100 mM NaCl, 10 mM $MgCl_2$, 20 mM HEPES), by filtering the suspension through two layers of cheesecloth.

Binding curves for the under study compounds were derived indirectly from competition experiments

against 0.5 nM $^3H$-pirenzepine labelling the muscarinic receptors of the cerebral cortex. 1 ml of the homogenate was incubated for 45 min at 30° C in the presence of a marker ligand and different concentrations of the cold ligand, conditions under which equilibrium was reached as determined by appropriate association experiments. The incubation was terminated by centrifugation (12,000 rpm for 3 min) at room temperature using an Eppendorf microcentrifuge. The resultant pellet was washed twice with 1.5 ml saline to remove the free radioactivity and it was allowed to drain for some hours. The tips of the tubes containing the pellet were cut off and 200 μl of tissue solubilizer (Lumasolve, Lumac) were added and left to stand overnight. Radioactivity was then counted after addition of 4 ml of liquid scintillation mixture (Dimilume/Toluene 1:10 v:v, Packard)

Assays were carried out in triplicate or quadruplicate and the non-specific binding was defined as the radioactivity bound or entrapped in the pellet when the incubation medium contained 1μM atropine sulphate. Non-specific binding avaraged less than 30%. $K_D$ values (dissociation constants) were obtained by non-linear regression analysis on the basis of a one binding site model with TOPFIT-pharmacokinetic programme package (G. HEINZEL, "Pharmacokinetics during drug development: data analysis and evaluation techniques" Eds. G. BOLZER and J.M. VAN ROSSUM; p. 207, G. Fisher, New York, 1982) after correction for the radioligand occupancy shift according to the equation: $K_D = IC_{50}/1 + {}^*C/{}^*K_D$, where $^*C$ and $^*K_D$ represent the concentration and the dissociation constant of the radioligand used, respectively.

The following table I shows the obtained results:

TABLE I

Antimuscarinic effect. Dissociation constants ($K_D$) for $^3H$-pirenzepine binding

| Compound | $K_D$ (nM) |
|---|---|
| 1 | 7 |
| 2 | 7.5 |
| 4 | 17 |
| 14 | 15 |
| 15 | 1.8 |
| 24 | 38 |
| 28 | 32 |
| 34 | 4.4 |
| 35 | 12 |
| 43 | 3.6 |
| 45 | 1.1 |
| 62 | 30 |
| 64 | 3.4 |
| 65 | 2 |

ANTISPASMODIC ACTIVITY

Antispasmodic activity of the compounds was tested on isolated guinea pig ileum with bethanechol as a spasmogen according to the method as described in Edinburgh Staff ("Pharmacological Experiments on Isolated Preparations" Second edition, Churchill Livingstone, Edimburgh, 1974).

Guinea pigs (450-55 g, Dunkin Hartley) were sacrificed by cervical dislocation, and a 2 cm piece of terminal ileum was rapidly excised. The tissue was mounted in a 10 ml organ bath containing Tyrode solution of the following composition: (mM) NaCl 137; KCl 2.68; $CaCl_2$ 1.82; $NaHCO_3$ 5.9; $MgCl_2$ 1; $NaH_2PO_4$ 0.42; glucose 5.6. Temperature was 37°C, resting tension 800 mg. Contractions were induced by cumulative addition of bethanechol (0.3-30 μM), each concentration being left in contact until maximal response was observed. Antagonists were added to the bath 60 min before repeating agonist stimulation.

The shift of bethanechol concentration-response curve induced by antagonists, dose-ratio (DR), was calculated by a computer programme (Tallarida, R. S., and Murray, R. B. "Manual of Pharmacologic Calculations with Computer Programs" Springer Verlag, New York, 1981) after verifying parallelism.

$K_B$ (dissociation constant) was estimated by linear regression analysis as:

$$K_B = \frac{[B]}{DR - 1},$$

where [B] represents the concentration of the antagonist under study.

The results are reported in the following table II:

TABLE II

Antispasmodic activity. Dissociation constants ($K_B$) for bethanechol induced contractions in the guinea pig ileum

| Compound | $K_B$ (nM) |
|---|---|
| 1 | 1.8 |
| 2 | 6.5 |
| 4 | 18 |
| 14 | 17 |
| 15 | 1 |
| 24 | 13 |
| 28 | 8.5 |
| 34 | 1.5 |
| 35 | 2.7 |
| 43 | 1.5 |
| 45 | 0.5 |
| 62 | 20 |
| 64 | 1 |
| 65 | 1.5 |

According to a further feature of the present invention there are provided pharmaceutical compositions comprising as active ingredient at least one compound of formula (I), as hereinbefore defined, or a physiologically compatible acid addition salt thereof in association with a pharmaceutical carrier or excipient. For pharmaceutical administration the compounds of general formula (I) and their physiologically compatible acid addition salts may be incorporated into the conventional pharmaceutical preparations in either solid liquid form. The compositions may, for example, be presented in a form suitable for oral, rectal or parenteral administration. Preferred forms include, for example, capsules, tablets, coated tablets, freeze-dried vials, suppositories and oral drops.

The active ingredient may be incorporated in excipients or carrier conventionally used in pharmaceutical compositions such as, for example, talc, gum arabic, lactose, gelatine, magnesium stearate, corn starch, aqueous or non-aqueous vehicles, polyvinylpirrolidone, mannitol, semisynthetic glicerides of fatty acids, sorbitol, propylene glycol, citric acid, sodium citrate.

The compositions are advantageously formulated at dosage units, each dosage unit being adapted to supply a single dose of the active ingredient. Each dosage unit may conveniently contain from 5 mg to 500 mg and preferably from 10 mg to 100 mg.

The following examples illustrate some of the new compounds according to the present invention; these examples are not to be considered in any way limitative of the scope of the invention itself:

Example 1

1-Phenyl-1-Cyclohexyl-3-amino-propanol

a) A solution of 1-phenyl-1-cyclohexyl-3-chloro-propanol (32 g) and potassium phthalimide (23.5 g) in DMF (100 ml) was heated at 100°C for 3 hours. The reaction mixture was cooled at room temperature , evaporated to dryness and dissolved between water and diethyl ether. The organic layer was then evaporated to dryness and from the crude residue, the pure 1-phenyl-1-cyclohexyl-3-(N-phthaloyl)-amino-propanol was obtained after crystallization from diisopropyl ether. 27.5 g, M.p. 102-104°C.

b) 1-phenyl-1-cyclohexyl-3-(N-phthaloyl)-amino-propanol (6.6 g) in 95% ethanol (60 ml) was refluxed for 1 hour in the presence of 85% hydrazine hydrate (2.25 g). The reaction mixture was evaporated to dryness, dissolved in water and basified with 10% NaOH. The oily product which separated was extracted into diethyl ether; the organic solution was evaporated to dryness and the pure title compound was obtained after crystallization from diisopropyl ether. 3.7 g, M. p. 84-87°C.

Example 2

N-[(2-hydroxy-2-cyclohexyl-2-phenyl)-ethyl]-piperazine

a) Cyclohexyl magnesium chloride (2.6 M in diethyl ether, 100 ml) was slowly dropped into a well stirred solution of N-benzyl-N′-phenacyl-piperazine (35 g) in anhydrous diethyl ether (350 ml). After 1 hour stirring, a NaCl solution in water (50 ml)was cautiously added to the reaction mixture under cooling. A grey solid was obtained and collected by filtration. The intermediate N-benzyl-N′-[(2-hydroxy-2-cyclohexyl-2-phenyl)-ethyl]-piperazine was obtained after purification of the crude residue by flash chromatography technique (eluent toluene-ethylacetate 70:30) on silica gel. 15.9 g, M.p. 253-254°C (dec.),as hydrochloride salt.

b) A solution of N-benzyl-N′-[(2-hydroxy-2-cyclohexyl-2-phenyl)-ethyl]-piperazine, dihydrocloride (5.9 g) in methanol (200 ml) and water (20 ml) was hydrogenated at room temperature and pressure in the presence of 10% Pd/C (0.8 g). When the theoretical amount of hydrogen was taken up, the reaction mixture was filtered and evaporated to dryness. A white waxy solid resulted from which the title compound was obtained after trituration with diethyl ether. 3.6 g, M.p. 243-246°C (dec.).

Example 3

(2-Aminoethyl )-α-cyclohexyl-cyclohexyl carboxylate

a) α-Cyclohexyl-cyclohexyl carbonyl chloride (3.2 g) in THF (20 ml) was added dropwise to a solution of N-CBZ-ethanol amine (2.8 g) and $NEt_3$ (1.6 g) in THF (30 ml). The reaction mixture was stirred overnight at room temperature, and evaporated to dryness. The residue was taken up into diethyl ether and washed with 5% HCl, water, 5% NaOH and water. From the organic layer, after evaporation to dryness, 5 g of the crude 2-(N-CBZ-amino)-ethyl-α-cyclohexyl-cyclohexyl carboxylate were obtained as thick oil.

b) The above intermediate (3 g) in 95% ethanol (30 ml) was hydrogenated at room temperature and pressure in the presence of 10% Pd/C and 25% HCl in ethanol (1.2 ml). When the theoretical amount of $H_2$ was taken up, the reaction mixture was filtered and evaporated to give 2.3 g of the title compound as

hydrochloride salt. M.p. 68-72° C (dec.).

Example 4

2-(Piperidin-4-yl)-1,1-diphenyl-ethanol

a) A solution of ethyl-(N-benzyl-piperidin-4-yl) acetate (3 g) in anhydrous THF was slowly dropped at 10° C into a 2 M phenyl magnesium chloride solution in THF (64 ml). The reaction mixture was stirred 5 hours at room temperature and the crude 2-(N-Benzyl-piperidin-4-yl)-1,1-diphenyl ethanol was extracted into ethyl acetate. After evaporation of the solvent and crystallization from diethyl ether 8.9 g of the above compound were obtained. M.p. 119-121° C.

b) This intermediate (2.15 g) was dissolved in methanol (24 ml) and hydrogenated at room temperature and pressure in the presence of 5% Pd/C. When the theoretical amount of $H_2$ was taken up, the reaction mixture was filtered and evaporated to dryness. From the residue, after crystallization from diethyl ether, 1.3 g of the title compound were obtained as a white solid. M.p. 277-281° C (dec.) (as hydrochloride salt).

Example 5

Benzeneacetic acid, α-phenyl-, 4-piperidinyl ester, hydrochloride

a) To a solution of benzeneacetic acid, α-phenyl-,chloride (30 g) in benzene (60 ml) 1-benzyl-4-hydroxy-piperidine (24.8 g) dissolved in benzene (60 ml)was slowly added with stirring. The reaction mixture was gently refluxed for additional 3 hours until a clear solution was obtained. Upon cooling benzeneacetic acid, -phenyl-, 1-benzyl-4-piperidinyl ester, hydrochloride cristallized and was collected by filtration. 48 g, M.p. 200-201° C.

b) A solution of benzeneacetic acid, α-phenyl-,1-benzyl-4-piperidinyl ester, hydrochloride (48 g) in ethanol was hydrogenated at room temperature and at atmospheric pressure in the presence of 10% Pd/C. The catalyst was then filtered off, the solvent removed under vacuum. The residual thick oil was triturated with diethyl ether to give 36.7 g of the title compound hydrochloride. M.p. 159-160° C. The free base was obtained by the usual methods and crystallized from diisopropyl ether, M.p. 82-84° C.

Example 6

Benzeneacetic acid, α-cyclopentyl-α-hydroxy,3-pyrrolidinyl ester

A solution of benzeneacetic acid, α-cyclopentyl-α-hydroxy-,1-benzyl-3-pyrrolidinyl ester, hydrochloride (2.7 g) in ethanol was hydrogenated at room temperature and pressure in the presence of 10% Pd/C to give 1.7 g of the title compound as a thick oil. M.p. 68° -70° C after liophylization. The free base was obtained by the usual methods and used without further purification.

Example 7

4-Amino-2,2-diphenylbutiramide

a) To a solution of 4-chloro-2,2-diphenylbutiramide (2.7 g) in acetonitrile (100 ml) was added IRA 400 resin loaded with sodium azide (1.9 g), prepared according to A. Hassner et al. (Angew. Chem. Int. Ed. Engl. 25, 478. 1986). After stirring at room temperature for 48 hours, the resin was removed by filtration and the reaction mixture was concentrated to an oil (2.5 g). The crude 4-azido-2,2-diphenylbutiramide was used as such for the next reaction.

IR (mjol): $cm^{-1}$ 2090, 1690 - 1660

b) 4-Azido-2,2-diphenylbutiramide (2,5 g) was dissolved in ethanol (120 ml) and hydrogenated at room temperature and pressure in the presence of 10% Pd.C (0.2 g). After filtration and concentration the crude product (1.8 g) was obtained. This product was purified by flash chromatography (eluent: $CH_2Cl_2$ - $CH_3OH$ - 32% $NH_4OH$ 80:20:2) on silica gel. 0.4 g of the title product were obtained.

IR (mjol): $cm^{-1}$ 3480 - 3100, 1690 - 1660

MS (C.I.): 255 m/e $[M + H]^+$

Hydrochloride (ethanol). M.p. 90° -92° C.

Example 8

α-Phenyl-α-(3-pyridin)-acetic acid, 4-piperidinyl ester

a) α-Phenyl-α-(3-pyridin)-acetic acid (2.5 g) and carbonildiimidazole (1.9 g) were added to anydrous dimethylformamide (10 ml) under stirring. After 10 minutes N-carbobenzyloxy-4-hydroxypiperidine (2.76 g) and 1,8-diazabicyclo[5.4.0]undec-7-ene (DBU) (1.78 g) were added. The reaction mixture was stirred at room temperature for 4 hours. After purification by flash chromatography (eluent: methylene chloride - ethylacetate 7:3) 3.8 g of α-phenyl-α-(3-pyridin)-acetic acid-(N-carbobenzyloxy)-4-piperidinyl ester were obtained as yellow oil.
IR (mjol): $cm^{-1}$ 1730, 1700

b) After hydrogenation of a solution of α-phenyl-α-(3-pyridin)-acetic acid-(N-carbobenzyloxy)-4-piperidinyl ester (3 g) in ethanol (100 ml) at room temperature and pressure in the presence of 10% Pd/C (0.3 g) 2.2 g of the title compound were obtained as yellow oil.
IR (mjol): $cm^{-1}$ 1730

Example 9

γ-Pentinoic acid, α-phenyl,4-piperidinyl ester

a) To a suspension of sodium amide (prepared from2.3 g of sodium) in 200 ml of liquid ammonia ethylphenylacetate (16.4 g) dissolved in diethyl ether (10 ml) was added. Propargylbromide dissolved in diethyl ether (20 ml) was then added at the same temperature. After stirring for 30 minutes, 100 ml of diethyl ether were added and the reaction mixture was stirred overnight allowing the evaporation of the ammonia. The reaction mixture was then decomposed by pouring into ice/water, acidified, and the ethereal layer separated. The concentration of this layer gave a crude oil which was distilled.α-Phenyl-γ-pentinoic acid ethyl ester distilled at 95°-100° C at 0,25 mm Hg. Yield 14.6 g.

b) α-Phenyl-γ-pentinoic acid was obtained by saponification of the corresponding ethyl ester with KOH in acqueos ethanol. M.p. 89°-90° C.

c) α-Phenyl-γ-pentinoic acid was esterified with N-carbobenzyloxy-4-hydroxy-piperidine in the presence of carbonildiimidazole and DBU in dimethylformamide according to the example 8 a). α-Phenyl-γ-pentinoic acid-(N-carbobenzyloxy)-4-piperidinyl ester was obtained as an oil.
IR (mjol): $cm^{-1}$ 2120, 1730, 1700.

d) To a solution of α-phenyl-γ-pentinoic acid-(N-carbobenzyloxy)-4-piperidinyl ester (3 g) in anydrous acetonitrile (50 ml) were added sodium iodide (3.4 g) and trimethylchlorosilane (1.66 g)dissolved in acetonitrile (10 ml) under stirring at room temperature. Stirring was continued overnight, then a solution of hydrochloric acid in methanol was added, the whole was stirred at room temperature for 30 minutes and then concentrated to dryness. The residue was taken up into water, washed with ethyl acetate, basified and extracted into ethyl acetate. 1.85 g of the desired product was obtained as an oil.
IR (mjol): $cm^{-1}$2130, 1730

Example 10

N-guanyl-Noratropine

(Compound 1)

A solution of noratropine hydrochloride (7 g) and cyanamide (1.8 g) in water (40 ml) was heated at 100° C for 4 hours. The crude residue was purified by chromatography on silica gel (eluent: methylene dichloride -methanol - water 80:20:2) to afford the desired compound as hydrochloride salt. 2.9 g, M.p. 75°-79° C (dec.) (from diethyl ether).
M S (C.I.): 318 m/e $[M + H]^+$

Analysis

| | | | | | | | |
|---|---|---|---|---|---|---|---|
| | Found % | C | 56.94 | H | 6.98 | N | 11.70 |
| $C_{17}H_{24}ClN_3O_3$ | | | | | | | |
| | Calc. % | C | 57.69 | H | 6.83 | N | 11.87 |

The following N-guanyl derivatives were also prepared utilizing the procedure above described:

(-)N-guanyl-Norscopolamine

(Compound 2)
Hydrochloride salt (diethyl ether). M.p. 160-161°C (dec.).
M S (C.I.): 332 m/e $[M + H]^+$

Analysis

| | | | | | | | |
|---|---|---|---|---|---|---|---|
| $C_{17}H_{22}ClN_3O_4$ | Found % | C | 57.62 | H | 6.45 | N | 11.79 |
| | Calc. % | C | 58.04 | H | 6.30 | N | 11.94 |

N-guanyl-Noranisotropine

(Compound 3)
Hydrochloride salt (diethyl ether). M.p. 76-78°C (dec.)
M S (C.I.): 296 m/e $[M + H]^+$

Analysis

| | | | | | | | |
|---|---|---|---|---|---|---|---|
| $C_{16}H_{30}ClN_3O_2$ | Found % | C | 55.50 | H | 9.54 | N | 11.94 |
| | Calc. % | C | 57.90 | H | 9.11 | N | 12.66 |

N-guanyl-N'-[(2-phenyl-2-cyclohexyl-2-hydroxy)-ethyl]-piperazine

(Compound 4)
Dihydrochloride salt (acetonitrile). M.p. 185°C (dec.)
M S (C.I.): 331 m/e $[M + H]^+$

Analysis

| | | | | | | | |
|---|---|---|---|---|---|---|---|
| $C_{19}H_{32}Cl_2N_4O$ | Found % | C | 55.88 | H | 8.14 | N | 13.51 |
| | Calc. % | C | 56.57 | H | 7.99 | N | 13.89 |

N-Guanyl-N′-[(2,2-diphenyl-2-hydroxy)-ethyl]-piperazine

(Compound 5)
Dihydrochloride salt (acetonitrile) M.p. 215°C (dec.)
MS (C.I.): 326 m/e $[M + H]^+$

Analysis

| | | | | | | | |
|---|---|---|---|---|---|---|---|
| $C_{19}H_{26}Cl_2N_4O$ | Found % | C | 56.81 | H | 6.80 | N | 14.51 |
| | Calc. % | C | 57.43 | H | 6.60 | N | 14.10 |

N-Guanyl-N′-[(2-phenyl-2-cyclohexyl-2-hydroxy)-ethyl]-homopiperazine

(Compound 6)
Dihydrochloride salt (acetone) M.p. 185°C (dec.)
MS (C.I.): 346 m/e $[M + H]^+$

Analysis

| | | | | | | | |
|---|---|---|---|---|---|---|---|
| $C_{20}H_{34}Cl_2N_4O$ | Found % | C | 56.74 | H | 8.12 | N | 14.13 |
| | Calc. % | C | 57.55 | H | 8.21 | N | 13.42 |

N-Guanyl-N′-[(2-phenyl-2-cyclopentyl-2-hydroxy)-ethyl]-piperazine

(Compound 7)
Dihydrochloride salt (diethyl ether) M.p. 180°C (dec.)
MS (C.I.): 318 m/e $[M + H]^+$

Analysis

| | | | | | | | |
|---|---|---|---|---|---|---|---|
| $C_{18}H_{30}Cl_2N_4O$ | Found % | C | 54.83 | H | 7.59 | N | 14.26 |
| | Calc. % | C | 55.52 | H | 7.77 | N | 14.39 |

N-Guanyl-N′-[(2-phenyl-2-hydroxy-3-ethyl)-pentyl]-piperazine

(Compound 8)
Dihydrochloride salt (acetone) M.p. 245°C (dec.)
MS (C.I.) 320 m/e $[M + H]^+$

Analysis

| | | | | | | | |
|---|---|---|---|---|---|---|---|
| $C_{18}H_{32}Cl_2N_4O$ | Found % | C | 54.44 | H | 8.12 | N | 14.56 |
| | Calc. % | C | 55.24 | H | 8.24 | N | 14.32 |

N-Guanyl-N′-[(3-phenyl-3-hydroxy-3-cyclohexyl)-propyl]-piperazine

(Compound 9)
Dihydrochloride salt (Ethanol) M.p. 265°C (dec.)
MS (C.I.): 346 m/e $[M + H]^+$

Analysis

| | | | | | | | |
|---|---|---|---|---|---|---|---|
| $C_{20}H_{34}Cl_2N_4O$ | Found % | C | 56.98 | H | 8.32 | N | 13.91 |
| | Calc. % | C | 57.55 | H | 8.21 | N | 13.42 |

N-Guanyl-N′-[(2hydroxy-2-phenyl-2-cyclohexyl)-ethyl]-2-ketopiperazine

(Compound 10)
Hydrochloride salt (diethyl ether)M.p. 115°C (dec.)
MS (C.I.): 346 m/e $[M + H]^+$

Analysis

| | | | | | | | |
|---|---|---|---|---|---|---|---|
| $C_{19}H_{29}ClN_4O_2$ | Found % | C | 58.83 | H | 7.76 | N | 15.61 |
| | Calc. % | C | 59.91 | H | 7.67 | N | 14.71 |

Example 11

1-Phenyl-1-cyclohexyl-(N-guanyl)-3-amino-propanol

(Compound 11)

A solution of 1-phenyl-1-cyclohexyl-3-amino-propanol (0,5 g) and 2-methyl-2-thiopseudourea sulfate (0.3 g) in methanol (5 ml) and water (1 ml) was heated at 60°C for 6 hours. The solution was evaporated to dryness and the residue, after crystallization from acetonitrile, furnished 0.35 g of the title compound. M.p. 260°C (dec.).
MS (C.I.): 276 m/e $[M + H]^+$

Analysis

| | | | | | | | |
|---|---|---|---|---|---|---|---|
| $C_{32}H_{52}N_6O_6S$ | Found % | C | 58.98 | H | 8.18 | N | 12.53 |
| | Calc. % | C | 59.23 | H | 8.08 | N | 12.45 |

By utilising the suitable amino derivative and following the above procedure, the following N-guanyl derivatives were prepared:

[N-guanyl-(2-amino ethyl)]-α-cyclohexyl-cyclohexyl carboxylate

(Compound 12)
Sulphate salt (acetonitrile). M.p. 198-200°C (dec.).
M S (C.I.): 296 m/e $[M + H]^+$

Analysis

| | | | | | | | |
|---|---|---|---|---|---|---|---|
| $C_{32}H_{60}N_6O_8S$ | Found % | C | 55.45 | H | 8.83 | N | 12.04 |
| | Calc. % | C | 55.78 | H | 8.77 | N | 12.20 |

Example 12

N-nitro-guanyl-noratropine

A solution of noratropine (5 g) and S-methyl-3-nitro-thiopseudourea (2.3 g) in methanol (50 ml) and methylene dichloride (50 ml) was stirred at room temperature. After two days stirring the reaction mixture was evaporated to dryness and the pure title compound was obtained from the residue after chromatography on silica gel (eluent $CH_2Cl_2$ - MeOH 95:5). 1.85 g. M. p. 94-95°C.
According to this procedure and utilizing the suitable amino derivative the following compounds were prepared:
-1,1-Diphenyl-2-(N-nitro guanyl-piperidin-4-yl)ethanol. M.p. 218-222°C.
-(-)-N-nitro guanyl-norscopolamine. M.p. 106-109°C.
Benzeneacetic acid, α-phenyl,1-(N-nitroguanyl)-3-piperidinyl ester. M.p. 68-70°C.
Benzeneacetic acid, α-phenyl-,1-(N-nitroguanyl)-4-piperidinyl ester
M.p. 165-168°C.
Benzeneacetic acid, α-cyclopentyl-α-hydroxy-,1-(N-nitroganyl)-3-pyrrolidinyl ester. M.p. 175-178°C.
Piperidine,4-(diphenylmethylene)-,1-(N-nitroguanyl). M.p. 188-190°C.
Butiramide,2,2-diphenyl-4-(N-nitroguanidino). M.p. 225-228°C.

Example 13

Benzeneacetic acid, α-phenyl,1-(N-guanyl-piperidin-3-yl)ester, hydrochloride

(Compound 13)

A solution of benzeneacetic acid, α-phenyl,1-(N-nitroganyl))-3-piperidinyl ester (1,9 g) in formic acid (20 ml) was dropped into a well stirred suspension of 10% Pd/C (1 g) in formic acid (2 ml). The suspension was further

stirred for 4 hours at 40°C, filtered and evaporated to dryness. 20% HCl in ethanol (20 ml) was added to the residue and the resulting solution was evaporated to dryness. The hydrochloride of the title compound was obtained as a white solid after crystallization from diethyl ether and acetone. 1.2 g. Hydrochloride salt (diethyl ether) M.p. 218-220°C (dec.).
MS (C.I.): 338 m/e M + H $^+$

Analysis

| | | | | | | | |
|---|---|---|---|---|---|---|---|
| $C_{20}H_{24}ClN_3O_2$ | Found % | C | 62.80 | H | 6.52 | N | 11.14 |
| | Calc. % | C | 64.25 | H | 6.47 | N | 11.24 |

Analoguosly the following guanidino derivatives were obtained:

N-guanyl-noratropine

(Compound 1)
Hydrochloride salt (diethyl ether). M.p. 78-80°C
M S (C.I.): 318 m/e $[M + H]^+$

Analysis

| | | | | | | | |
|---|---|---|---|---|---|---|---|
| $C_{17}H_{24}ClN_3O_3$ | Found % | C | 57.12 | H | 6.99 | N | 11.45 |
| | Calc. % | C | 57.69 | H | 6.83 | N | 11.82 |

(-)-N-guanyl-norscopolamine

(Compound 2)
Hydrochloride salt (diethyl ether).M.p. 159-161°C (dec.)
M S (C.I.): 332m/e $[M + H]^+$

Analysis

| | | | | | | | |
|---|---|---|---|---|---|---|---|
| $C_{17}H_{22}ClN_3O_4$ | Found % | C | 57.77 | H | 6.43 | N | 11.88 |
| | Calc. % | C | 58.04 | H | 6.30 | N | 11.94 |

Benzeneacetic acid,α-phenyl-,1-guanyl-4-piperidinyl ester

(Compound 14)
Hydrochloride salt (toluene). M.p. 160-161°C.
M S (C.I.): 338 m/e $[M + H]^+$

Analysis

| | | | | | | | | | |
|---|---|---|---|---|---|---|---|---|---|
| $C_{20}H_{24}ClN_3O_2$ | Found % | C | 63.36 | H | 6.45 | Cl | 9.52 | N | 11.03 |
| | Calc. % | C | 64.25 | H | 6.47 | Cl | 9.48 | N | 11.24 |

Benzeneacetic acid, α-cyclopentyl-α-hydroxy-,1-guanyl-3-pyrrolidinyl ester

(Compound 15)
Formate salt (diethyl ether). M.p. 170-173° C.
MS (C.I.) : 378 m/e $[M + H]^+$

Analysis

| | | | | | | | |
|---|---|---|---|---|---|---|---|
| $C_{19}H_{27}N_3O_5$ | Found % | C | 59.52 | H | 7.01 | N | 10.95 |
| | Calc. % | C | 60.46 | H | 7.21 | N | 11.13 |

Piperidine,4-(diphenylmethylene)-,1-guanyl

(Compound 16)
Hydrochloride salt ($H_2O$). M.p. > 270°C.
MS (C.I.): 292 m/e $[M + H]^+$

Analysis

| | | | | | | | |
|---|---|---|---|---|---|---|---|
| $C_{19}H_{22}ClN_3$ | Found % | C | 69.35 | H | 6.85 | N | 12.51 |
| | Calc. % | C | 69.60 | H | 6.76 | N | 12.82 |

Butiramide,2,2-diphenyl-4-guanidino

(Compound 17)
Formate salt (diethyl ether) M.p. 68-70°C
MS (C.I.): 297 m/e $[M + H]^+$

Analysis

| | | | | | | | |
|---|---|---|---|---|---|---|---|
| $C_{18}H_{22}N_4O_3$ | Found % | C | 62.57 | H | 6.54 | N | 16.12 |
| | Calc. % | C | 63.14 | H | 6.48 | N | 16.36 |

Benzeneacetic acid,α-cyclohexyl-,1-guanyl-4-piperidinyl ester

(Compound 18)
Formate salt (diethyl ether). M.p. 205-210°C
MS (C.I.): 344 m/e $[M + H]^+$

Analysis

| | | | | | | | |
|---|---|---|---|---|---|---|---|
| $C_{21}H_{31}N_3O_4$ | Found % | C | 63.32 | H | 7.99 | N | 10.51 |
| | Calc. % | C | 64.75 | H | 8.02 | N | 10.79 |

Benzeneacetic acid,α-isopropyl-,1-guanyl-4-piperidinyl ester

(Compound 19)
Formate salt (diethyl ether)M.p. 185-190°C
MS (C.I.) : 304 m/e $[M + H]^+$

Analysis

| | | | | | | | |
|---|---|---|---|---|---|---|---|
| $C_{18}H_{27}N_3O_4$ | Found % | C | 61.21 | H | 7.71 | N | 11.93 |
| | Calc. % | C | 61.87 | H | 7.79 | N | 12.03 |

Benzeneacetic acid,α-butyl-,1-guanyl-4-piperidinyl ester

(Compound 20)
Formate salt (diethyl ether). M.p. 174-175°C
MS (C.I.): 318 m/e $[M + H]^+$

Analysis

| | | | | | | | |
|---|---|---|---|---|---|---|---|
| $C_{19}H_{29}N_3O_4$ | Found % | C | 62.03 | H | 7.91 | N | 11.28 |
| | Calc. % | C | 62.78 | H | 8.04 | N | 11.56 |

Benzeneacetic acid,α-phenyl-α-hydroxy-,1-guanyl-4-piperidinyl ester

(Compound 21)
Formate salt (diethyl ether). M.p. 215-217°C
MS (C.I.): 354 m/e $[M + H]^+$

Analysis

| | | | | | | | |
|---|---|---|---|---|---|---|---|
| $C_{21}H_{25}N_3O_5$ | Found % | C | 62.35 | H | 6.33 | N | 10.36 |
| | Calc. % | C | 63.14 | H | 6.31 | N | 10.52 |

Benzeneacetic acid,α-phenyl-,1-guanyl-3-pyrrolidinyl ester

(Compound 22)
Formate salt (diethyl ether) M.p. 176°-177°C
MS (C.I.): 324 m/e $[M + H]^+$

Analysis

| | | | | | | | |
|---|---|---|---|---|---|---|---|
| $C_{20}H_{23}N_3O_4$ | Found % | C | 64.48 | H | 6.21 | N | 11.29 |
| | Calc. % | C | 65.02 | H | 6.27 | N | 11.38 |

Benzeneacetic acid,α-phenyl-,4-guanyl-1-piperazinyl amide

(Compound 23)
Formate salt (diethyl ether) M.p. 243-245°C
MS (C.I.): 323 m/e $[M + H]^+$

Analysis

| | | | | | | | |
|---|---|---|---|---|---|---|---|
| $C_{20}H_{24}N_4O_3$ | Found % | C | 63.86 | H | 6.34 | N | 14.93 |
| | Calc. % | C | 65.19 | H | 6.56 | N | 15.21 |

Example 14

2-[(N-guanyl-piperidin-4-yl)]-1,1-diphenyl-ethanol

(Compound 24)

A solution of 2-(N-nitroguanyl-piperidin-4-yl)-1,1-diphenyl ethanol (0.4 g) in methanol (20 ml) was hydrogenated at room temperature and pressure in the presence of 10% Pd/C (0.15 g) and glacial acetic acid (0.06 ml). When the theoretical amount of hydrogen was taken up, the reaction mixture was filtered and evaporated to dryness. The title compound was obtained as a white solid after crystallization from acetone. 0.3 g. M.p. 235-238°C (dec.).
MS (C.I.): 324 m/e $[M + H]^+$

Analysis

| | | | | | | | |
|---|---|---|---|---|---|---|---|
| $C_{20}H_{25}N_3O$ | Found % | C | 68.88 | H | 7.81 | N | 10.65 |
| | Calc. % | C | 68.90 | H | 7.62 | N | 10.96 |

The following substituted guanyl derivatives were also prepared utilizing standard procedures from the appropriate precursors.

Benzeneacetic acid,α-phenyl-,1-(N-methyl)-guanyl-4-piperidinyl ester

(Compound 25)

Hydrochloride salt M.p. 80-82°C (Freeze-dried)
MS (C.I.) : 352 m/e $[M + H]^+$

Analysis

| | | | | | | | |
|---|---|---|---|---|---|---|---|
| $C_{21}H_{26}ClN_3O_2$ | Found % | C | 64.03 | H | 6.57 | N | 10.47 |
| | Calc. % | C | 65.02 | H | 6.75 | N | 10.83 |

Benzeneacetic acid,α-phenyl-,1-(N,N-dimethyl)-guanyl-4-piperidinyl ester

(Compound 26)
Hydrochloride salt M.p. 65-70°C (Freeze-dried)
MS (C.I.) : 366 m/e [M + H]$^+$

Analysis

| | | | | | | | |
|---|---|---|---|---|---|---|---|
| $C_{22}H_{28}ClN_3O_2$ | Found % | C | 64.51 | H | 6.89 | N | 10.30 |
| | Calc. % | C | 65.74 | H | 7.02 | N | 10.45 |

Example 15

Benzeneacetic acid,α-phenyl,1-[N-methyl-(thiocarbamoyl)]-4-piperidinyl ester

To a solution of benzeneacetic acid,α-phenyl,4-piperidinyl ester (2.0 g) in tetrahydrofuran (40 ml) was added dropwise under stirring a solution of methyl isothiocyanate (0.5 g) in the same solvent. After stirring for additional three hours the reaction mixture was concentrated under vacuum. The residue was dissolved in diisopropyl ether and left overnigth. The title compound (2.4 g) was recovered by filtration. M.p. 118°-120°C.

According to this procedure and utilizing the suitable amino derivative, the following compounds were prepared:
Piperidine,4-(diphenylmethylene)-,1-[N-methyl-(thiocarbamoyl)] M.p. 160-161°C.
-(-)-N-methyl-N′-norscopolamine-thiourea. M.p. 100-104°C (dec.).
N-methyl-N′-[(3-hydroxy-3-phenyl-3-cyclohexyl)-propyl]-thiourea. M.p. 98-101°C.
1-[N-methyl-(thiocarbamoyl)]-4-[(2-hydroxy-2-phenyl-2-cyclohexyl)-ethyl]-piperazine. M.p. 174-176°C.

Example 16

Benzeneacetic acid,α-phenyl,1-[N-methyl-(iminomethyl)]-4-piperidinyl ester hydrochloride

(Compound 27)

A solution of benzeneacetic acid,α-phenyl,1-[N-methyl-(thiocarbamoyl)-4-piperidinyl ester (0.5 g) in methylene dichloride (20 ml) was allowed to react with Ni/Raney (3.0 g) under stirring. After 3 hours the reaction mixture was filtered, 20 % ethanolic hydrochloric acid was added (congo red), then, after evaporation of the solvents under vacuum, a solid was isolated. After lyophilization the title compound weighed 0.32 g, M.p. 65-70°C.
M S (C.I.): 337 m/e [M + H]$^+$

Analysis

| | | | | | | | |
|---|---|---|---|---|---|---|---|
| $C_{21}H_{25}ClN_2O_2$ | Found % | C | 66.82 | H | 7.03 | N | 7.14 |
| | Calc. % | C | 67.64 | H | 6.76 | N | 7.51 |

By utilizing the suitable N-alkyl-(thiocarbamoyl) derivative and following the above procedure, the following N-alkyl-(iminomethyl) derivatives were prepared:

Piperidine,4-(diphenylmethylene)-,1-[N-methyl-(iminomethyl)]

(Compound 28)
Hydrochloride salt (acetonitrile) M.p.>270°C.
M S (C.I.): 291 m/e [M + H]+

Analysis

| | | | | | | | |
|---|---|---|---|---|---|---|---|
| $C_{20}H_{23}ClN_2$ | Found % | C | 72.93 | H | 7.14 | N | 8.62 |
| | Calc. % | C | 73.49 | H | 7.09 | N | 8.57 |

-(-)-N-methyl-iminomethyl-norscopolamine

(Compound 29)
Hydrochloride salt (diethyl ether) M.p. 132-133°C (dec.)
M S (C.I.): 331 m/e [M + H]+

Analysis

| | | | | | | | |
|---|---|---|---|---|---|---|---|
| $C_{18}H_{23}ClN_2O_4$ | Found % | C | 58.11 | H | 6.48 | N | 7.45 |
| | Calc. % | C | 58.94 | H | 6.32 | N | 7.64 |

(-)-[(N-benzyl)iminomethyl]-norscopolamine

(Compound 30)
Hydrochloride salt (acetone). M.p. 93-95°C (dec.)
M S (C.I.): 407 m/e [M + H]+

Analysis

| | | | | | | | |
|---|---|---|---|---|---|---|---|
| $C_{24}H_{27}ClN_2O_4$ | Found % | C | 64.78 | H | 6.18 | N | 6.11 |
| | Calc. % | C | 65.07 | H | 6.14 | N | 6.32 |

Benzeneacetic acid,α-phenyl-,1-(N-n-hexyliminomethyl)-4-piperidinyl ester

(Compound 31)
Hydrochloride salt M.p. 65-70°C (Freeze-dried).
MS (C.I.): 407 m/e [M + H]+

Analysis

| | | | | | | | |
|---|---|---|---|---|---|---|---|
| $C_{26}H_{35}ClN_2O_2$ | Found % | C | 69.85 | H | 7.99 | N | 6.21 |
| | Calc. % | C | 70.49 | H | 7.96 | N | 6.32 |

N-methyl-N′-[(3-hydroxy-3-phenyl-3-cyclohexyl)-propyl]-formamidine

(Compound 32)
Hydrochloride salt (diethyl ether). M.p. 77-80°C (dec.)
MS (C.I.): 275 m/e $[M + H]^+$

Analysis

| | | | | | | | |
|---|---|---|---|---|---|---|---|
| $C_{17}H_{22}ClN_2O$ | Found % | C | 65.02 | H | 8.88 | N | 8.90 |
| | Calc. % | C | 65.68 | H | 8.76 | N | 9.01 |

1-[(N-methyl)-iminomethyl]-4-[(2-hydroxy-2-phenyl-2-cyclohexyl)-ethyl]-piperazine

(Compound 33)
Hydrochloride salt (diethyl ether) M.p. 250-252°C.
MS (C.I.): 330 m/e $[M + H]^+$

Analysis

| | | | | | | | |
|---|---|---|---|---|---|---|---|
| $C_{20}H_{33}Cl_2N_3O$ | Found % | C | 59.73 | H | 8.22 | N | 10.28 |
| | Calc. % | C | 59.69 | H | 8.27 | N | 10.44 |

Example 17

Benzeneacetic acid,α-phenyl,1-(iminomethyl)-4-piperidinyl ester, hydrochloride

(Compound 34)

To a solution of benzeneacetic acid,α-phenyl,4-piperidinyl ester (8.0 g) in a 1:1 mixture of ethyl acetate and acetonitrile (150 ml) freshly prepared ethyl formimidate hydrochloride (3.5 g) was added portionwise under stirring. Stirring was continued for additional 30 minutes, then the reaction mixture was filtered and allowed to stand. The crystallized title compound was recovered by filtration. 5.2 g, M.p. 182-184°C.
M S (C.I.): 323 m/e $[M + H]^+$

Analysis

| | | | | | | | | | |
|---|---|---|---|---|---|---|---|---|---|
| $C_{20}H_{23}ClN_2O_2$ | Found % | C | 66.40 | H | 6.40 | Cl | 10.12 | N | 8.04 |
| | Calc. % | C | 66.93 | H | 6.46 | Cl | 9.88 | N | 7.81 |

The following compounds have been prepared according to the above described procedure:

N-iminomethyl-N'-[(2-hydroxy-2-phenyl-2-cyclohexyl)-ethyl]-piperazine

(Compound35)
Hydrochloride salt (acetonitrile). M.p. 218-220°C (dec.)
M S (C.I.): 316 m/e [M + H]$^+$

Analysis

| | | | | | | | |
|---|---|---|---|---|---|---|---|
| $C_{19}H_{30}ClN_3O$ | Found % | C | 64.78 | H | 8.63 | N | 11.85 |
| | Calc. % | C | 64.84 | H | 8.59 | N | 11.94 |

Benzeneacetic acid,α-cyclohexyl-α-hydroxy-,1-(iminomethyl)-4-piperidinyl ester

(Compound 36)
Hydrochloride salt (Ethanol) M.p. 268-269°C
MS (C.I.): 345 m/e [M + H]$^+$

Analysis

| | | | | | | | |
|---|---|---|---|---|---|---|---|
| $C_{20}H_{29}ClN_2O_3$ | Found % | C | 62.97 | H | 7.70 | N | 7.32 |
| | Calc. % | C | 63.06 | H | 7.67 | N | 7.36 |

Benzeneacetic acid,α-phenyl-,1-(iminomethyl)-3-pyrrolidinyl ester

(Compound 37)
Hydrochloride salt (ethylacetate-acetonitrile) M.p. 110-113°C.
MS (C.I.): 309 m/e [M + H]$^+$

Analysis

| | | | | | | | |
|---|---|---|---|---|---|---|---|
| $C_{19}H_{21}ClN_2O_2$ | Found % | C | 65.41 | H | 6.33 | N | 7.96 |
| | Calc. % | C | 66.17 | H | 6.17 | N | 8.12 |

Benzeneacetic acid,α-cyclohexyl-,1-(iminomethyl)-4-piperidinyl-ester

(Compound 38)
Hydrochloride salt (ethyl acetate-acetonitrile) M.p. 234-235°C
MS (C.I.): 329 m/e $[M + H]^+$

Analysis

| | | | | | | | |
|---|---|---|---|---|---|---|---|
| $C_{20}H_{29}ClN_2O_2$ | Found % | C | 65.56 | H | 8.04 | N | 7.61 |
| | Calc. % | C | 65.83 | H | 8.01 | N | 7.67 |

Benzeneacetic acid,α-phenyl,-8-(iminomethyl)-8-azabicyclo-[3.2.1]-oct-3-yl ester

(Compound 39)
Hydrochloride salt (Ethanol) M.p. 274-275°C.
MS (C.I.): 349 m/e $[M + H]^+$

Analysis

| | | | | | | | |
|---|---|---|---|---|---|---|---|
| $C_{22}H_{25}ClN_2O_2$ | Found % | C | 68.15 | H | 6.59 | N | 7.24 |
| | Calc. % | C | 68.65 | H | 6.55 | N | 7.28 |

Benzeneacetic acid,α-isopropyl-,1-(iminomethyl)-4-piperidinyl ester

(Compound 40)
Hydrochloride salt (ethyl acetate) M.p. 195-196°C
MS (C.I.): 289 m/e $[M + H]^+$

Analysis

| | | | | | | | |
|---|---|---|---|---|---|---|---|
| $C_{17}H_{25}ClN_2O_2$ | Found % | C | 62.45 | H | 7.61 | N | 8.58 |
| | Calc. % | C | 62.85 | H | 7.76 | N | 8.62 |

Benzeneacetic acid,α-phenyl-α-hydroxy-,1-(iminomethyl)-4-piperidinyl ester

(Compound 41)
Hydrochloride salt (ethanol-ethyl acetate) M.p. 262-264°C.
MS (C.I.): 339 m/e $[M + H]^+$

Analysis

| | | | | | | | |
|---|---|---|---|---|---|---|---|
| $C_{20}H_{23}ClN_2O_3$ | Found % | C | 63.65 | H | 6.21 | N | 7.39 |
| | Calc. % | C | 64.08 | H | 6.18 | N | 7.47 |

Benzeneacetic acid,α-methyl-α-phenyl-,1-(iminomethyl)-4-piperidinyl ester

(Compound 42)
Hydrochloride salt (ethyl acetate - acetonitrile) M.p. 172-173°C.
MS (C.I.): 337 m/e $[M + H]^+$

Analysis

| | | | | | | | |
|---|---|---|---|---|---|---|---|
| $C_{21}H_{25}ClN_2O_2$ | Found % | C | 67.01 | H | 6.82 | N | 7.43 |
| | Calc. % | C | 67.64 | H | 6.76 | N | 7.51 |

Example 18

(-)-(N-iminomethyl)-norscopolamine, hydrochloride

(Compound 43)

To a solution of (-)-norscopolamine (2.0 g)in absolute ethanol (15 ml) freshly prepared ethyl formimidate hydrochloride (0.76 g) was added under stirring. Stirring was continued overnight then the reaction mixture was filtered and concentrated under vacuum. The crude residue was purified by chromatography on silica gel (eluent: methylene dichloride-methanol - water 80:20:2°) to afford the title compound as hydrochloride salt. 0.7 g, M.p. 80-90°C
(after lyophilization) MS (C.I.): 317 m/e $[M + H]^+$

Analysis

| | | | | | | | | | |
|---|---|---|---|---|---|---|---|---|---|
| $C_{17}H_{21}ClN_2O_4$ | Found % | C | 57.10 | H | 6.12 | Cl | 9.87 | N | 7.85 |
| | Calc. % | C | 57.87 | H | 6.00 | Cl | 10.05 | N | 7.94 |

By utilizing the suitable amino derivative and following the above procedures, the following N-(iminomethyl) derivatives were prepared:

Benzeneacetic acid,α-phenyl,1-(iminomethyl)-3-piperidinyl ester

(Compound 44)
Hydrochloride salt (toluene) M.p. 207-208°C
M S (C.I.): 323 m/e $[M + H]^+$

Analysis

| | | | | | | | | | |
|---|---|---|---|---|---|---|---|---|---|
| $C_{20}H_{23}ClN_2O_2$ | Found % | C | 68.42 | H | 6.58 | Cl | 9.53 | N | 7.46 |
| | Calc. % | C | 66.93 | H | 6.46 | Cl | 9.88 | N | 7.81 |

N-(iminomethyl)-noratropine

(Compound 45)
Hydrochloride salt (acetonitrile-diethyl ether) M.p. 202-203°C (dec.)
M S (C.I.): 303 m/e $[M + H]^+$

Analysis

| | | | | | | | |
|---|---|---|---|---|---|---|---|
| $C_{17}H_{23}ClN_2O_3$ | Found % | C | 59.88 | H | 6.99 | N | 8.13 |
| | Calc. % | C | 60.25 | H | 6.84 | N | 8.26 |

N-(iminomethyl)-N'-[(2,2-diphenyl-2-hydroxy)-ethyl]-piperazine

(Compound 46)
Dihydrochloride salt (acetonitrile) M.p. 270°C
MS (C.I.): 311 m/e $[M + H]^+$

Analysis

| | | | | | | | |
|---|---|---|---|---|---|---|---|
| $C_{19}H_{23}N_3O \cdot 2HCl$ | Found % | C | 65.40 | H | 7.01 | N | 12.05 |
| | Calc. % | C | 65.98 | H | 6.99 | N | 12.15 |

N-(iminomethyl)-N'-[(2-phenyl-2-cyclohexyl-2-hydroxy)-ethyl]-homopiperazine

(Compound 47)
Dihydrochloride salt (ethyl acetate) M.p. 231°C
MS (C.I.): 331 m/e $[M + H]^+$

Analysis

| | | | | | | | |
|---|---|---|---|---|---|---|---|
| $C_{20}H_{33}Cl_2N_3O$ | Found % | C | 59.48 | H | 8.32 | N | 10.33 |
| | Calc. % | C | 59.70 | H | 8.27 | N | 10.44 |

N-(iminomethyl)-N'-[(2-phenyl-2-cyclopentyl-2-hydroxy)-ethyl]-piperazine

(Compound 48)
Dihydrochloride salt (diethyl ether) M.p.>270°C
MS (C.I.) : 303 m/e $[M + H]^+$

Analysis

| | | | | | | | |
|---|---|---|---|---|---|---|---|
| $C_{18}H_{29}Cl_2N_3O$ | Found % | C | 57.55 | H | 7.88 | N | 11.13 |
| | Calc. % | C | 57.75 | H | 7.81 | N | 11.22 |

N-(iminomethyl)-N'-[(3-phenyl-3-hydroxy-3-cyclohexyl)-propyl]-piperazine

(Compound 49)
Dihydrochloride salt (acetonitrile) M.p. 210°C
MS (C.I.) : 331 m/e $[M + H]^+$

Analysis

| | | | | | | | |
|---|---|---|---|---|---|---|---|
| $C_{20}H_{33}Cl_2N_3O$ | Found % | C | 59.12 | H | 8.31 | N | 10.36 |
| | Calc. % | C | 59.75 | H | 8.27 | N | 10.44 |

N-(iminomethyl)-N'-[(2-phenyl-2-hydroxy-3-ethyl)-pentyl]-piperazine

(Compound 50)
Dihydrochloride salt (acetone) M.p. 225°C
MS (C.I.) : 305 m/e $[M + H]^+$

Analysis

| | | | | | | | |
|---|---|---|---|---|---|---|---|
| $C_{18}H_{31}Cl_2N_3O$ | Found % | C | 56.92 | H | 8.41 | N | 11.05 |
| | Calc. % | C | 57.44 | H | 8.30 | N | 11.16 |

N-(iminomethyl)-N'-[(2-hydroxy-2-phenyl-2-cyclohexyl)-ethyl]-2-ketopiperazine

(Compound 51)
Hydrochloride salt (acetonitrile) M.p. 188°C
MS (C.I.): 331 m/e $[M + H]^+$

Analysis

| | | | | | | | |
|---|---|---|---|---|---|---|---|
| $C_{19}H_{28}ClN_3O_2$ | Found % | C | 61.93 | H | 7.79 | N | 11.37 |
| | Calc. % | C | 62.36 | H | 7.71 | N | 11.48 |

[N-(iminomethyl)]-2-aminoethyl-α-cyclohexyl- cyclohexyl carboxylate

(Compound 52)
Hydrochloride salt (diethyl ether) M.p. 96-100°C (dec.)
M S (C.I.): 281 m/e $[M + H]^+$

Analysis

| | | | | | | | |
|---|---|---|---|---|---|---|---|
| $C_{16}H_{29}ClN_2O_2$ | Found % | C | 60.03 | H | 9.41 | N | 8.50 |
| | Calc. % | C | 60.64 | H | 9.22 | N | 8.84 |

N-(3-hydroxy-3-phenyl-3-cyclohexyl)-propyl-formamidine

(Compound 53)
Hydrochloride salt (acetonitrile-diethyl ether) M.p. 175-178°C (dec.)
M S (C.I.): 261 m/e $[M + H]^+$

Analysis

| | | | | | | | |
|---|---|---|---|---|---|---|---|
| $C_{17}H_{25}ClN_2O$ | Found % | C | 64.09 | H | 8.29 | N | 9.12 |
| | Calc.% | C | 64.96 | H | 8.18 | N | 9.47 |

Example 19

Benzeneacetic acid,α-phenyl,1-[1′-(N-methylimino)ethyl]-4-piperidinyl ester, hydrochloride

(Compound 54)

To a suspension of benzeneacetic acid,α-phenyl,4-piperidinyl ester, hydrochloride (1.0 g) in absolute ethanol (10 ml) was added a solution of N-methyl-phenylacetimidate in the same solvent (5 ml). The resulting solution was stirred at room temperature for 2 hours, then concentrated to dryness. The crude material was taken up into hot ethyl acetate from which 0.7 g of the pure title compound are recovered after crystallization.
M.p. 183-185°C.
M S (C.I.): 351 m/e $[M + H]^+$

Analysis

| | | | | | | | | | |
|---|---|---|---|---|---|---|---|---|---|
| $C_{22}H_{27}ClN_2O_2$ | Found % | C | 67.93 | H | 7.10 | Cl | 9.31 | N | 7.20 |
| | Calc. % | C | 68.29 | H | 7.03 | CL | 9.16 | N | 7.24 |

Example 20

Benzeneacetic acid,α-phenyl,1[1'-(imino)ethyl]-4-piperidinyl ester, hydrochloride

(Compound 55)

To a solution of benzeneacetic acid,α-phenyl,4-piperidinyl ester (1.5 g) in absolute ethanol (15 ml) ethylacetimidate hydrochloride (0.7 g) was added under stirring. The reaction mixture was kept stirred for further 3 hours then concentrated to dryness. The crude material was suspended in hot toluene and filtered. After three days the filtrate gave 0.4 g of the title compound, M.p. 179-182°C.
M S (C.I.): 337 m/e$[M + H]^+$

Analysis

| | | | | | | | | | |
|---|---|---|---|---|---|---|---|---|---|
| $C_{21}H_{25}ClN_2O_2$ | Found % | C | 67.01 | H | 6.80 | Cl | 9.63 | N | 7.39 |
| | Calc. % | C | 67.64 | H | 6.76 | Cl | 9.51 | N | 7.51 |

The following compounds have been prepared according to the above described procedure starting from the appropriate amino compound:

Benzeneacetic acid, α-butyl-,1-[1'-(imino)ethyl]-4-piperidinyl ester

(Compound 56)
Hydrochloride salt M.p. 62-65°C (Freeze-dried)
MS (C.I.): 317 m/e $[M + H]^+$

Analysis

| | | | | | | | |
|---|---|---|---|---|---|---|---|
| $C_{19}H_{29}ClN_2O_2$ | Found % | C | 64.18 | H | 8.41 | N | 7.74 |
| | Calc. % | C | 64.67 | H | 8.28 | N | 7.93 |

Benzeneacetic acid, α-phenyl-,8-[1'-(imino)ethyl]-8-azabicyclo[3.2.1]-oct-3-yl ester

(Compound 57)
Hydrochloride salt (isopropanol) M.p.>270°C
MS (C.I.) : 363 m/e $[M + H]^+$

Analysis

| | | | | | | | |
|---|---|---|---|---|---|---|---|
| $C_{23}H_{27}ClN_2O_2$ | Found % | C | 69.04 | H | 6.91 | N | 6.92 |
| | Calc. % | C | 69.24 | H | 6.82 | N | 7.02 |

3-Pyridineacetic acid, α-phenyl-,1-[1′-(imino)ethyl]-4-piperidinyl ester

(Compound 58)
Dihydrochloride salt M.p. 80-81°C (Freeze-dried)
MS (C.I.): 338 m/e [M + H]$^+$

Analysis

| | | | | | | | |
|---|---|---|---|---|---|---|---|
| $C_{20}H_{25}Cl_2N_3O_2$ | Found % | C | 57.81 | H | 6.23 | N | 10.03 |
| | Calc. % | C | 58.54 | H | 6.14 | N | 10.24 |

Benzeneacetic acid, α-cyclohexyl-α-hydroxy-,1-[1′-(imino)ethyl]-4-piperidinyl ester

(Compound 59)
Hydrochloride salt (ethyl acetate) M.p. 228-230°C
MS (C.I.): 359 m/e [M + H]$^+$

Analysis

| | | | | | | | |
|---|---|---|---|---|---|---|---|
| $C_{21}H_{31}ClN_2O_3$ | Found % | C | 63.11 | H | 7.99 | N | 7.01 |
| | Calc. % | C | 63.86 | H | 7.91 | N | 7.09 |

Benzeneacetic acid, α-phenyl-,1-[1′-(imino)propyl]-4-piperidinyl ester

(Compound 60)
Hydrochloride salt (diethyl ether) M.p. 186-188°C
MS (C.I.) : 351 m/e [M + H]$^+$

Analysis

| | | | | | | | |
|---|---|---|---|---|---|---|---|
| $C_{22}H_{27}ClN_2O_2$ | Found % | C | 67.25 | H | 7.19 | N | 7.09 |
| | Calc. % | C | 68.29 | H | 7.03 | N | 7.24 |

γ-Pentinoic acid, α-phenyl-1,-[1′-(imino)ethyl]-4-piperidinyl ester

(Compound 61)
Hydrochloride salt M.p. 70-72°C (Freeze-dried)
MS (C.I.) : 299 m/e [M + H]$^+$

Analysis

| | | | | | | | |
|---|---|---|---|---|---|---|---|
| $C_{18}H_{23}ClN_2O_2$ | Found % | C | 63.85 | H | 7.03 | N | 8.09 |
| | Calc. % | C | 64.56 | H | 6.92 | N | 8.37 |

N-(iminoethyl)-N′-[(2-hydroxy-2-phenyl-2-cyclohexyl)ethyl]-piperazine

(Compound 62)
Hydrochloride salt (acetonitrile) M.p. 202°C (dec.)
M S (C.I.): 330 m/e $[M + H]^+$

Analysis

| | | | | | | | |
|---|---|---|---|---|---|---|---|
| $C_{20}H_{33}Cl_2N_3O$ | Found % | C | 59.81 | H | 8.30 | N | 10.46 |
| | Calc. % | C | 59.69 | H | 8.27 | N | 10.44 |

Example 21

N-(3-hydroxy-3-phenyl-3-cyclohexyl)-propyl-acetamidine

(Compound 63)

A solution of 1-phenyl-1-cyclohexyl-3-amino-propanol (1 g) and ethyl acetimidate hydrochloride (0.5 g) in ethanol was stirred overnight at room temperature. The reaction mixture was evaporated to dryness and from the residue, after column chromatography on silica gel (eluents : methylene dichloride - methanol - water 80:20:2) the pure title compound was obtained as hydrochloride salt. 0.6 g, M.p. 100-104°C (dec.).
M S (C.I.): 275 m/e $[M + H]^+$

Analysis

| | | | | | | | |
|---|---|---|---|---|---|---|---|
| $C_{17}H_{27}ClN_2O$ | Found % | C | 64.87 | H | 8.95 | N | 8.83 |
| | Calc. % | C | 65.68 | H | 8.76 | N | 9.01 |

The following compounds have been similarly prepared:

N-iminoethyl-noratropine

(Compound 64)
Hydrochloride salt (diethyl ether) M.p. 205-206°C (dec.)
M S (C.I.): 317 m/e [M + H]+

Analysis

| | | | | | | | |
|---|---|---|---|---|---|---|---|
| $C_{18}H_{25}ClN_2O_3$ | Found % | C | 60.89 | H | 7.25 | N | 7.55 |
| | Calc. % | C | 61.27 | H | 7.14 | N | 7.94 |

(-)-N-iminoethyl-norscopolamine

(Compound 65)
Hydrochloride salt (diethyl ether) M.p. 182-186°C (dec.)
M S (C.I.): 331 m/e [M + H]+

Analysis

| | | | | | | | |
|---|---|---|---|---|---|---|---|
| $C_{18}H_{23}ClN_2O_4$ | Found% | C | 58.10 | H | 6.48 | N | 7.44 |
| | Calc.% | C | 58.94 | H | 6.32 | N | 7.64 |

N-(iminoethyl)-N′-[(2,2-diphenyl-2-hydroxy)-ethyl]-piperazine

(Compound 66)
Dihydrochloride salt (Ethanol) M.p. 235°C
MS (C.I.): 325 m/e [M + H]+

Analysis

| | | | | | | | |
|---|---|---|---|---|---|---|---|
| $C_{20}H_{27}Cl_2N_3O$ | Found % | C | 60.15 | H | 6.91 | N | 10.55 |
| | Calc. % | C | 60.61 | H | 6.87 | N | 10.60 |

N-(iminoethyl)-N′-[(2-phenyl-2-cyclohexyl-2-hydroxy)-ethyl]-homopiperazine

(Compound 67)
Dihydrochloride salt (acetone) M.p. 187°C
MS (C.I.): 345 m/e [M + H]+

Analysis

| | | | | | | | |
|---|---|---|---|---|---|---|---|
| $C_{21}H_{35}Cl_2N_3O$ | Found % | C | 60.10 | H | 8.53 | N | 10.02 |
| | Calc. % | C | 60.57 | H | 8.47 | N | 10.09 |

N-(iminoethyl)-N'-[(2-phenyl-2-cyclopentyl-2-hydroxy)-ethyl]-piperazine

(Compound 68)
Dihydrochloride salt (Ethanol) M.p. 205°C
MS (C.I.): 317 m/e $[M + H]^+$

Analysis

| | | | | | | | |
|---|---|---|---|---|---|---|---|
| $C_{19}H_{31}Cl_2N_3O$ | Found % | C | 57.98 | H | 8.12 | N | 10.75 |
| | Calc. % | C | 58.76 | H | 8.05 | N | 10.82 |

N-(iminoethyl)-N'-[(2-phenyl-2-hydroxy-3-ethyl)-pentyl]-piperazine

(Compound 69)
Dihydrochloride salt (diethyl ether) M.p. 190°C (dec.)
MS (C.I.): 319 m/e $[M + H]^+$

Analysis

| | | | | | | | |
|---|---|---|---|---|---|---|---|
| $C_{19}H_{33}Cl_2N_3O$ | Found % | C | 57.89 | H | 8.60 | N | 10.65 |
| | Calc. % | C | 58.46 | H | 8.52 | N | 10.76 |

N-(iminoethyl)-N'-[(3-phenyl-3-cyclohexyl-3-hydroxy)-propyl]-piperazine

(Compound 70)
Dihydrochloride salt (acetonitrile) M.p. 265°C
MS (C.I.): 345 m/e $[M + H]^+$

Analysis

| | | | | | | | |
|---|---|---|---|---|---|---|---|
| $C_{21}H_{35}Cl_2N_3O$ | Found % | C | 60.21 | H | 8.55 | N | 9.95 |
| | Calc. % | C | 60.57 | H | 8.47 | N | 10.09 |

N-(iminoethyl)-N′-[(2-hydroxy-2-phenyl-2-cyclohexyl)-ethyl]-2-ketopiperazine

(Compound 71)
Hydrochloride (diethyl ether) M.p. 140°C (dec.)
MS (C.I.): 345 m/e $[M + H]^+$

Analysis

| | | | | | | | |
|---|---|---|---|---|---|---|---|
| $C_{20}H_{30}ClN_3O_2$ | Found % | C | 62.76 | H | 7.99 | N | 11.00 |
| | Calc. % | C | 63.22 | H | 7.96 | N | 11.06 |

Benzeneacetic acid, α-methyl-α-phenyl-,1-[1′-(imino)ethyl]-4-piperidinyl ester

(Compound 72)
Hydrochloride salt (ethyl acetate) M.p. 176-177°C
MS (C.I.) : 351 m/e $[M + H]^+$

Analysis

| | | | | | | | |
|---|---|---|---|---|---|---|---|
| $C_{22}H_{27}ClN_2O_2$ | Found % | C | 67.85 | H | 7.09 | N | 7.17 |
| | Calc. % | C | 68.29 | H | 7.03 | N | 7.24 |

Example 22

Benzeneacetic acid,α-phenyl,4-guanyl-1-piperidinylamide, hydrochloride salt

(Compound 73)

a) To a solution of phenylacetic acid,α-phenyl-chloride (10.4 g) in tetrahydrofurane (100 ml) were added 4-cyanopiperidine (5.45 g) and triethylamine (5.9 g). The resulting suspension was stirred overnight at room temperature, then evaporated to dryness. The raw material was taken up in diethylether and washed with acidic water, and then with water until neutrality and finally concentrated to dryness. Pure benzeneacetic acid,α-phenyl,4-cyano-1-piperidinyl amide was obtained by crystallization from ethanol. 10.5 g . M.p. 170-173°C.

b) An intimate mixture of benzeneacetic acid,α-phenyl,4-cyano-1-piperidinylamide (4 g) and ammonium thiocyanate (4 g) was heated to 180 °C and the molten mass was kept at that temperature for 6 hours. The

solid obtained was taken up into diluted hydrochloric acid, then washed with ethylacetate. The organic layer was washed, dried, and then concentrated to dryness. The raw material was purified by flash chromatography technique on silicagel (eluent Acetone-ethylacetate-water 60:30:10). The almost pure product was collected and then crystallized from acetonitrile-ethylacetate 1:2. 1.2 g M.p.>260°C. Hydrochloride salt.
M S (C.I.): 322 m/e [M + H]$^+$

Analysis

| | | | | | | | |
|---|---|---|---|---|---|---|---|
| $C_{26}H_{28}ClN_3O$ | Found % | C | 66.81 | H | 6.80 | N | 11.68 |
| | Calc. % | C | 67.12 | H | 6.76 | N | 11.74 |

Example 23

Benzeneacetic acid,α-phenyl,4-(N-phenyl) guanyl-1-piperidinylamide hydrochloride salt

(Compound 74)

To a mixture of benzeneacetic acid,α-phenyl-4-cyano-1-piperidinylamide (2 g), aniline (0.61 g) and tetrachloroethane (10 ml) was added portionwise aluminium trichloride (0.87 g). After the initial exothermic reaction had subsided, the reaction mixture was heated to 150°C, kept for 90 min. and then evaporated to dryness. The residue was taken up in water where crystallization took place. Recrystallization from ethanol gave pure title compound as hydrochloride salt (0.5 g). M.p.>260°C.
M S (C.I). : 398 m/e [M + H]$^+$

Analysis

| | | | | | | | |
|---|---|---|---|---|---|---|---|
| $C_{20}H_{24}ClN_3O$ | Found % | C | 71.27 | H | 6.59 | N | 9.57 |
| | Calc. % | C | 71.95 | H | 6.50 | N | 6.68 |

The following not limitative examples of pharmaceutical compositions according to the invention are reported:

Example 24

Tablets
-active ingredient 20 mg
- lactose 247 mg
- corn starch 30 mg
- magnesium stearate 3 mg

Method of preparation: the active ingredient, lactose and corn starch were mixed and homogeneously moistened with water. After screening of the moist mass and drying in a tray drier, the mixture was again passed through a screen and magnesium stearate was added. Then the mixture was pressed into tablets weighing 300 mg each. Each tablet contains 20 mg of active ingredient.

Example 25

Capsules
- active ingredient 20 mg
- lactose 178 mg
- magnesium stearate 2 mg

Method of preparation: the active ingredient was mixed with the auxiliary products, and the mixture was passed through a screen and mixed homogeneously in a suitable device. The resulting mixture was filled into hard gelatine capsules (200 mg per capsule); each capsule contains 20 mg of active ingredient.

Example 26

Freeze-dried vials
- active ingredient 10 mg
- mannitol 50 mg

Method of preparation: the active ingredient and mannitol were dissolved in an appropriate amount of water for injection. The resulting solution was filtered and filled into vials under sterile conditions. The vials were freeze-dried and stopped with a suitable closure.

Example 27

Suppositories
- active ingredient 50 mg
- semisynthetic glicerides of fatty acids 750 mg

Method of preparation: the semisynthetic glicerides of fatty acids were melted and the active ingredient was added while stirring homogeneously. After cooling at a proper temperature the mass was poured into preformed moulds for suppositories weighing 800 mg each. Each suppository contains 50 mg of active ingredient.

Example 28

Oral drops
- active ingredient 10 mg
- sorbitol 350 mg
- propylene glycol 200 mg
- citric acid 1 mg
- sodium citrate 3 mg
- demineralized water q.s. 1 ml

Method of preparation: the active ingredient, citric acid and sodium citrate were dissolved in a mixture of a proper amount of water and propylene glycol. Then sorbitol was added and the final solution was filtered. The solution contains 1% of active ingredient and is administered by using a proper dropper.

**Claims**

1. Compounds of general formula (I)

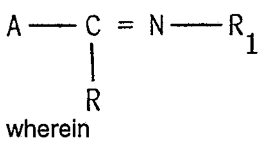

(I)

wherein
R represents hydrogen atom, $C_{1-5}$ alkyl, aryl, an amino group optionally substituted by one or two $C_{1-5}$ alkyl;
$R_1$ represents hydrogen atom, $C_{1-8}$ alkyl, aralkyl;
A represents
$C_{1-5}$ alkyl substituted by 2 or 3 radicals, which may be identical or different from each other, selected from aryl, cycloalkyl, carboxamide;
or a saturated 6-membered heterocyclic ring containing one nitrogen atom N-substituted by $COR_2$ where $R_2$ is a methyl substituted by 2 or 3 radicals, which may be identical or different from each other, selected

from aryl, cycloalkyl, hydroxy;
or

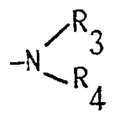

where $R_3$ is hydrogen atom and $R_4$ is $C_{1-5}$ alkyl substitued by 2 or 3 radicals, which may be identical or different from each other, selected from aryl, cycloalkyl, carboxamide, or by hydroxy optionally esterified with a cycloalkylcarboxylic acid, or $R_3$ and $R_4$, together with the nitrogen atom to which they are attached, form a saturated 5- to 7-membered heterocyclic ring, optionally containing one or two nitrogen atoms or an endocyclic carboxamido group optionally comprising in its inside $-CH_2-CH_2-$ or

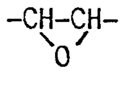 ,

being the heterocyclic ring substituted by a $C_{1-5}$ alkyl substituted in turn by 2 or 3 radicals, which may be identical or different from each other, selected from $C_{1-5}$ alkyl, aryl, cycloalkyl or hydroxy, or by arylalkylene, or by $-OR_5$ where $R_5$ is $-OCR_6$ where $R_6$ is methyl substituted by 2 or 3 radicals, which may be identical or different from each other, selected from a saturated or unsaturated $C_{1-5}$ alkyl, aryl, cycloalkyl, hydroxy $C_{1-5}$ alkyl, hydroxy, or unsaturated 6-membered heterocyclic ring, provided that at least one between R and A is amino group or

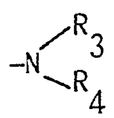

tautomers thereof and acid addition salts of the aforesaid compounds.

2. Physiologically compatible acid addition salts of compounds of formula (I) according to claim 1.

3. Salts according to claim 2 in which the physiologically compatible acids are hydrochloric, hydrobromic, sulphuric or methansulphonic acid.

4. Compounds according to claim 1 wherein R is a hydrogen atom, a $C_{1-5}$ alkyl or an unsubstituted amino group, $R_1$ is a hydrogen atom, a $C_{1-5}$ alkyl and A is a radical selected from noratropine, norscopolamine, N-[(2-hydroxy-2-cyclohexyl-2-phenyl)-ethyl-]piperazine, N-[(2-hydroxy-2-cyclohexyl-2-phenyl)-ethyl]-homopiperazine, N-[(2-hydroxy-2,2-diphenyl)-ethyl]-piperazine,benzeneacetic acid 4-phenyl-4-piperidinyl ester, benzeneacetic acid α-cyclohexyl-4-piperidinyl ester, benzeneacetic acid α-hydroxy-α-phenyl-4-piperidinyl ester or benzeneacetic acid α-methyl-α-phenyl-4-piperidinyl ester.

5. Physiologically compatible acid addition salts of compounds according to claim 4.

6. Salts according to claim 5 in which physiologically compatible acids are hydrochloric, hydrobromic, sulphuric or methansulphonic acid.

7. Pharmaceutical compositions comprising as active ingredient an effective amount of at least one compound of general formula (I) according to claim 1 or a tautomer or physiologically compatible acid addition salt thereof in association with pharmaceutical carriers or excipients.

8. Pharmaceutical compositions according to claim 7 for use as anti-muscarinic agents.

9. Pharmaceutical compositions according to claim 8 for the treatment of patients suffering from motility disorders of the gastrointestinal or urogenital tract and from peptic ulcer diseases.

10. Process for the preparation of compounds of general formula (I)

$$A-\underset{\displaystyle R}{\underset{|}{C}}=N-R_1 \quad (I)$$

wherein the various substituents are as defined in claim 1, characterized in that, when R is hydrogen and A is

EMI ID=59/1 HE=20 WI=20 TI=CHE

in which $R_3$ and $R_4$ are as above defined, a compound of general formula (II)

A—H (II)

in which A is as above defined, is reacted with an additional salt of a compound of formula (III)

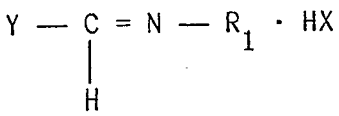

(III)

in which $R_1$ is as defined in claim 1, Y is a leaving group and HX is a mineral or organic acid, in a polar solvent at temperature between 10° and 70°.

11. Process for the preparation of the compounds of claim 1, characterized in that, in order to prepare compounds of general formula (I), in which R, A, $R_3$ and $R_4$ are as defined in claim 10, a thiourea of general formula (IV)

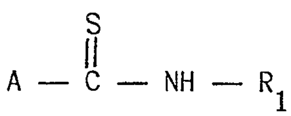

(IV)

in which A and $R_1$ are as above defined, is desulphurized by Raney-Nickel or $H_2O_2$ in an appropriate solvent at temperatures between 10° and 70° C.

12. Process for the preparation of the compounds according to claim 1, wherein R is $C_{1-5}$ alkyl and A is

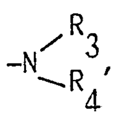

in which $R_3$ and $R_4$ are as defined in claim 1, characterized in that a compound of formula (II), A—H (II), in which A is as above defined, is reacted with a compound of formula (VI)

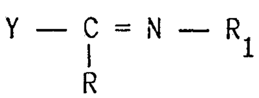

(VI)

in which R and Y are as above defined and $R_1$ is as defined in claim 1, in a polar solvent at temperatures between 10° and 70° C and that, when $R_1$ is a hydrogen atom, the compound of formula (II) is reacted with a cyano alkylderivative of formula (VII)

R—CN (VII)

in which R is as above defined, in the presence of a Lewis acid in an inert, high-boiling halogenated hydrocarbon or without solvent at temperatures between 50° and 200° C.

13. Process for the preparation of compounds of formula (I) of claim 1, characterized in that, when R is an amino group optionally substituted by one or two $C_{1-5}$ alkyl groups or by an aryl group and A is

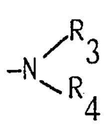

in which $R_3$ and $R_4$ are defined as in claim 1, and $R_1$ is hydrogen atom, a compound of formula (II), A—H (II), in the form of its acid addition salts with a mineral or organic acid of formula HX is reacted with cyanamide, optionally in the presence of a protic solvent at temperatures between 50° and 160° C or with a reactive compound of formula (VIII)

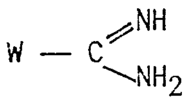

(VIII)

where W is a leaving group, in a polar solvent at temperatures between 20° and 100° C.

14. Process for the preparation of the compounds as defined in claim 13, characterized in that a nitroguanidine derivative of general formula (IX)

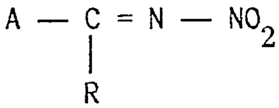

(IX)

in which A and R are as above defined, is reduced by hydrogen or a hydrogen donor in the presence of a suitable catalyst and optionally in the presence of a suitable solvent at temperatures between 10° and 100° C.

15. Process for the preparation of the compounds as defined in claim 14, characterized in that, when at least one between R and $R_1$ is different from hydrogen atom or from an unsubstituted amino group, a primary or secondary alkylamino derivative is reacted with a compound of general formula (XI)

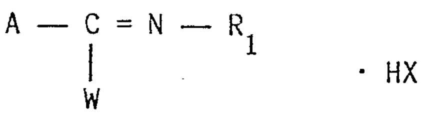

(XI)

where A, $R_1$, HX, W are as above defined, in the presence of a polar solvent at temperatures between 0° and 100° C.

16. Process for the preparation of the compounds of general formula (I) as defined in claim 1, characterized in that, when R is an amino group optionally substituted by one or two $C_{1-5}$ alkyl or by an aryl and A is $C_{1-5}$ alkyl substituted by 2 or 3 radicals, which may be identical or different from each other, selected from aryl, cycloalkyl, carboxamide, or it is a saturated 6-membered heterocyclic ring containing one nitrogen atom, N-substituted by $COR_2$ where $R_2$ is a methyl group substituted by 2 or 3 radicals, which may be identical or different from each other selected from aryl, cycloalkyl or hydroxy, a cyano derivative of general formula (XII)

A—CN (XII)

in which A is as above defined, is reacted with a substituted or unsubstituted ammonium salt of general formula (XIII)

R • HX (XIII)

where R and HX are as above defined, or with a substituted or unsubstituted thiourea of formula (XIV)

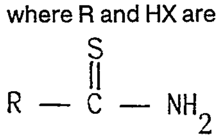

(XIV)

at temperatures between 50° and 200° C, or with a compound of formula (XV)

R—H (XV)

in which R is as above defined, in the presence of a Lewis acid in an inert, high-boiling, halogenated hydrocarbon at temperatures between 50° and 200° C, and if desired the compound of formula (XV) is reacted with an imidate or imidoyl of general formula (XVI)

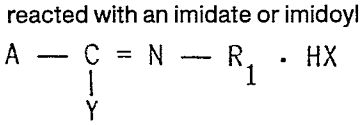

(XVI)

where A, Y, $R_1$ and HX are as above defined, in the presence of a solvent at temperatures between 0° and 80° C.

17. Process according to claim 10 or 12, characterized in that the leaving group is selected from halogen,

lower alkoxy, phenoxy or dichlorophosphoryl.

18. Process according to claim 12, characterized in that the compound of formula (II) and (VI) are used as free bases or as addition salts with mineral or organic acids of formula HX.

19. Process according to anyone of claims 13 and 15, characterized in that the leaving group, is 3,5-dimethylpirazol-1-yl, lower thioalkyl or sulphonyl.

20. Process according to claim 13, characterized in that the compound of formula (VIII) is reacted in the form of its addition salt with mineral or organic acid of formula HX.

21. Process according to claim 14, characterized in that in the reduction of the compound of formula (IX) the hydrogen donor is formic acid, acetic acid, ammonium formate, cyclohexene or cyclohexadiene, and the solvent is formic acid, methanol or ethanol.